# EUROPEAN PATENT APPLICATION

(11) **EP 1 790 296 A1**
(43) Date of publication of application: **30.05.2007**
(21) Application number: 05781037.6
(22) Date of filing: 29.08.2005
(51) Int. Cl.: A61B 17/11, A61B 17/00, A61F 2/06

(54) **AID FOR CORONARY ARTERY BYPASS OPERATION**

(30) Priority: 30.08.2004 JP 2004250444
(71) Applicant: JMS Co., Ltd., Hiroshima-shi, Hiroshima 730-8652 (JP)
(72) Inventor: KIMURA, Sosuke, 1840011 (JP); OMOTO, Tadashi, 1680063 (JP); HAYASHI, Shuro, JMS CO., LTD., Hiroshima-shi, Hiroshima 730-8652 (JP)
(74) Representative: Lorenz, Eduard
(86) International application number: PCT/JP2005/015626
(87) International publication number: WO 2006/025300

(57) **Abstract**

A slit 8 for insertion of the guide wire 3 is formed in the peripheral well of a tubular member 2 which is to be inserted into the left anterior descending branch. Athread 15 is tied around the peripheral wall of the tubular member 2 such that the knot 17 is placed on the peripheral well generally opposite to the slit 8. After the tybula member 2 is inserted into the left anterior descending branch with the help of the guide wire3, the guide wire 3 is pulled out. The thread 15 is lightly pulled to axially rotate the tubular member 2 such that the slit 8 is covered with the inner surface of the left anterior descending branch. After more than a half of a graft vessel is anastomosed to the periphery of an incision of the left anterior descending branch, the thread 15 is strongly pulled to pull out the tubular member 2. The graft vessel is completely anastomosed to the periphery of the incision.

## Description

### Technical Field

The present invention relates to a supporting device used in a coronary artery bypass surgery.

### Background Art

Over the surface of the cardiac muscle which is a constituent of the heart, coronary arteries are distributed to coronally extend from the origin at the aortic root of the upper part of the heart to surround the cardiac muscle. If arteriosclerosis causes stricture in such a coronary artery, supply of blood to the distal side of the coronary artery becomes insufficient, and accordingly, nutriment and oxygen supplied to the cardiac muscle are also insufficient, so that cardiac angina or myocardial infarct may be caused. A conventional treatment against such cardiac angina and myocardial infarct has been a coronary artery bypass surgery with a graft vessel. The coronary artery bypass surgery means such a surgery that an incision for anastomosis is formed in the distal side of the coronary artery beyond a stricture, and an end of a graft vessel anastomosed to, for example, the aortic sinus or an end of a graft vessel derived from an internal thoracic artery is anastomosed to the periphery of the incision, such that the blood flow detours around the stricture of the coronary artery to reach the distal side.

In recent years, the number of cases of the coronary artery bypass surgery carried out while keeping the heart pulsating with the view of reducing invasion has been increasing as disclosed in, for example, Patent Document 1. In the case of carrying out the coronary artery bypass surgery with the heart beating, blood is flowing through the coronary artery even in the presence of stricture. Bleeding from the incision formed in the coronary artery for anastomosis inhibits an operator from observing a surgery subject field, making an anastomosis operation significantly difficult. To prevent this problem, Patent Document 1 discloses that a ring-shape member with an opening at the center is placed such that a portion of the coronary artery to be anastomosed is within the opening, and is tightly pressed against the surface of the cardiac muscle. With this, the upstream of the coronary artery upper than the anastomosis site, i.e., the proximal side of the coronary artery, is pressed to be deformed such that the blood flow to the anastomosis site is interrupted, whereby bleeding by forming an incision is prevented.
[Patent Document 1] Japanese Laid-Open Patent Publication No. 10-234738 (Page 3, Page 4, Figure 2, and Figure 3)

### Disclosure of Invention

### Problems to be solved by the invention

However, interruption of the blood flow to the anastomosis site of the coronary artery as disclosed in Patent Document 1 entails interruption of the blood flow beyond the anastomosis site to the distal side of the coronary artery. As a result, supply of nutriment and oxygen to the cardiac muscle is also interrupted during the anastomosis operation although temporarily, so that an ischemic lesion can occur in the cardiac muscle.

The present invention was conceived in view of the above circumstances. An objective of the present invention is to prevent, in the operation of anastomosing a graft vessel to a coronary artery, bleeding from an incision formed in the coronary artery to secure observability in a surgery subject field, while maintaining the blood flow to the distal side of the coronary artery to avoid occurrence of a lesion in the cardiac muscle.

### Means for solving the problems

To achieve the above objective, according to the present invention, a coronary artery bypass surgery supporting device includes: a tubular member which is to be inserted into a coronary artery for allowing a blood flow from a proximal side to a distal side beyond an incision; a guide member which is inserted into the tubular member through an insertion section formed in a peripheral wall of the tubular member for guiding an end of the tubular member into the coronary artery; and a pull member extending from a portion of the tubular member generally opposite to the insertion section.

Specifically, the first invention is directed to a structure including: a flexible tubular member which is to be inserted into a coronary artery of a heart through an incision formed at a portion of the coronary artery for anastomosis of a graft vessel for allowing a blood flow from a proximal side to a distal side of the coronary artery beyond the incision, the tubular member having an insertion section in its peripheral wall; an elongated guide member which is inserted into the tubular member through the insertion section for guiding an end of the tubular member into the coronary artery for insertion of the tubular member into the coronary artery; and a pull member for pulling out the tubular member from the coronary artery through the incision, the pull member extending outside the tubular member from a portion of the tubular member generally opposite to the insertion section in a perimeter direction.

According to this structure, the guide member is inserted through the insertion section of the tubular member, a portion of the guide member protruding out of an opening of the tubular member at an end is inserted through the incision of the coronary artery into the inside of the coronary artery, and then, the end of the tubular member is moved along the guide member, whereby the end of the tubular member is guided into the inside of the coronary artery. With this feature, the tubular member can be inserted to a desired position in the pulsating coronary artery. Therefore, blood from the proximal side of the coronary artery can surely be transmitted through the tubular member to the distal side, so that the blood flow in the cardiac muscle is maintained.

While the tubular member is guided using the guide member in such a way, the non-insertion side of the guide member extends out from the incision of the coronary artery. Therefore, the insertion section of the tubular member through which the guide member is inserted faces the incision of the coronary artery to communicate with the outside of the coronary artery through the incision. In this situation, if the pull member, which extends from a portion of the tubular member generally opposite to the insertion section, is pulled lightly such that the tubular member does not come out of the incision of the coronary artery, the tubular member axially rotates so that the portion of the tubular member from which the pull member extends faces the incision of the coronary artery. Accordingly, the insertion section of the tubular member is shifted from the incision of the coronary artery to be covered with the inner surface of the coronary artery. As a result, the blood flowing through the tubular member is prevented from leaking through the insertion section, and even if some leaks, the leaking blood is within the coronary artery, so that the amount of blood leaking outside through the incision of the coronary artery is extremely small. Thus, the observability of the surgery subject field is improved.

Then, an end of a graft vessel is anastomosed to the periphery of the incision of the coronary artery. Before the whole perimeter of the graft vessel end is entirely anastomosed, the pull member is strongly pulled so that the tubular member is deformed to be flattened, and the flattened tubular member can be pulled out through a gap between the end of the graft vessel and the periphery of the incision. After the tubular member has been pulled out, the remaining part of the graft vessel is anastomosed to the periphery of the incision.

The second invention is directed to the first invention wherein a longitudinally intermediate part of the tubular member has an outside diameter smaller than an inside diameter of the coronary artery.

With this structure, a space can be generated between the periphery of the incision of the coronary artery and the outer surface of the tubular member. This space enables an easy anastomosis process such that a sewing needle for anastomosing the graft vessel to the periphery of the incision can be moved within the space.

The third invention is directed to the first or second invention wherein the tubular member has tight contact portions at both longitudinal ends. The tight contact portions have a diameter greater than an intermediate part of the tubular member such that an outer surface of the tight contact portions comes into tight contact with an inner surface of the coronary artery.

With this structure, the tight contact portion of the tubular member at the first end is positioned to be in tight contact with the inner surface of the proximal part of the coronary artery before the incision, so that the blood flowing through the coronary artery is prevented from flowing between the inner surface of the coronary artery and the outer surface of the tubular member. As a result, a blood flow into the tubular member is sufficiently secured. Meanwhile, the tight contact portion of the tubular member at the second end is positioned to be in tight contact with the inner surface of the distal part of the coronary artery beyond the incision, so that no blood flows through the outside of the tubular member from the distal side of the coronary artery to the longitudinally intermediate part. Thus, leakage of blood from the incision is suppressed.

The fourth invention is directed to the third invention wherein the both longitudinal ends of the tubular member have a diameter smaller than the outside diameter of the tight contact portions.

With this structure, the manipulation of inserting the tubular member into the coronary artery can easily be carried out.

### Effects of the invention

According to the first invention, the tubular member allowing the blood of the proximal side of the coronary artery before the incision to flow to the distal side is inserted into the coronary artery through the incision with the help of the guide member. Therefore, the tubular member can surely and readily be inserted to a desired position. As a result, the blood flow to the cardiac muscle is secured during a coronary artery bypass surgery, so that occurrence of a lesion in the cardiac muscle can be avoided. With the tubular member inserted in the coronary artery, the pull member which extends from a portion of the tubular member generally opposite to the insertion section is pulled such that the tubular member axially rotates, whereby the insertion section is covered with the inner surface of the coronary artery. With such a feature, the amount of blood leaking outside from the tubular member through the incision of the coronary artery is extremely small. Thus, the observability of the surgery subject field is secured.

According to the second invention, the outside diameter of the longitudinally intermediate part of the tubular member is smaller than the inside diameter of the coronary artery. Thus, a space for moving a sewing needle for anastomosis is formed between the periphery of the incision of the coronary artery and the outer surface of the tubular member. This space enables an easy anastomosis process, and the time required for the anastomosis process decreases.

According to the third invention, the tubular member has, at both longitudinal ends, tight contact portions which come into tight contact with an inner surface of the coronary artery. The blood flow into the tubular member can be sufficiently secured and transmitted to the cardiac muscle. The blood is prevented from flowing outside the tubular member from the distal side of the coronary artery and leaking through the incision, so that the observability of the surgery subject field is further improved.

According to the fourth invention, the longitudinal ends of the tubular member have a diameter smaller than the tight contact portions. Therefore, the tubular member can readily be inserted into the coronary artery.

### Brief Description of Drawings

[FIG.**1**] FIG.**1** is a side view showing a coronary artery bypass surgery supporting device according to embodiment 1 of the present invention.
[FIG.**2**] FIG.**2** is an enlarged partial cross-sectional view of a side of a guide wire.
[FIG.**3**] FIG.**3** illustrates the procedure of using the coronary artery bypass surgery supporting device according to embodiment 1. Part (a) shows that an end of a tubular member is inserted into the distal side of the left anterior descending branch. Part (b) shows that a guide wire is inserted into the proximal side of the left anterior descending branch.
[FIG.**4**] FIG.**4** illustrates the procedure of use as does FIG.**3**. Part (a) shows that an end of the tubular member is inserted into the proximal part of the left anterior descending branch. Part (b) shows the guide wire in the midst of being pulled out. Part (c) shows that a pull member is pulled after the guide wire has been pulled out.
[FIG.**5**] FIG.**5** illustrates the procedure of use as do FIG.**3** and FIG.**4**. Part (a) shows that part of a graft vessel is anastomosed. Part (b) shows the tubular member in the midst of being pulled out. Part (c) shows that the graft vessel is entirely anastomosed.
[FIG.**6**] FIG.**6** is a schematic view of a heart showing the location of the coronary artery and the graft vessel.
[FIG.**7**] FIG.**7** is a side view showing a coronary artery bypass surgery supporting device according to embodiment 2 of the present invention.
[FIG.**8**] FIG.**3** illustrates the procedure of using the coronary artery bypass surgery supporting device according to embodiment 2. Part (a) shows that an end of a guide wire is inserted into the distal side of the left anterior descending branch. Part (b) shows that an end of a tubular member is inserted into the distal side of the left anterior descending branch.
[FIG.**9**] FIG.**9** illustrates the procedure of use as does FIG.**8**. Part (a) shows that the guide wire is pulled out of the distal side of the left anterior descending branch. Part (b) shows that the guide wire is inserted into the proximal side of the left anterior descending branch. Part **(c)** shows that a pull member is pulled after the guide wire has been pulled out.
[FIG.10] FIG. **0** is a side view showing a coronary artery bypass surgery supporting device according to variation 1 of the embodiment.
[FIG.11] FIG.**11** is a side view showing a coronary artery bypass surgery supporting device according to variation 2 of the embodiment.
[FIG.12] FIG.**12** is a side view showing a coronary artery bypass surgery supporting device according to variation 3 of the embodiment.
[FIG.13] FIG.**13** illustrates the procedure of forming a slit in the tubular member. Part **(a)** is a side view. Part **(b)** is a cross-sectional view taken along line **A-A** of part (a).
[FIG.**14**] FIG.**14** is a partial cross-sectional view of the coronary artery bypass surgery supporting device according to embodiment 3 of the present invention.
[FIG.**15**] Part **(a)** of FIG.**15** is a side view of an insertion member seen from a side where no notch is formed. Part **(b)** of FIG.**15** is a side view of the insertion member seen from a side where a notch is formed.
[FIG.**16**] FIG.**16** shows a guide wire of embodiment 3.
[FIG.**17**] FIG.**17** illustrates the procedure of using the coronary artery bypass surgery supporting device according to embodiment 3. Part **(a)** shows that the ends of a tubular member are inserted into the left anterior descending branch. Part **(b)** shows that an end of a cylinder is inserted into the left anterior descending branch.
[FIG.**18**] FIG.**18** illustrates the procedure of use as does FIG.**17.** Part **(a)** shows that the tubular member and guide wire are inserted by a poll into the left anterior descending branch. Part (b) shows that the tubular member is entirely inserted into the left anterior descending branch.
[FIG.**19**] FIG.**19** illustrates the procedure of use as does FIG.**17** showing the guide wire in the midst of being pulled out.

### Description of Reference Numerals

- [0022] 1: Coronary artery bypass surgery supporting device
- 2: Tubular member
- 3: Guide wire (guide member)
- 4: Pull member
- 6: Tight contact portion
- 8: Slit (insertion section)
- A: Coronary artery
- E: Incision
- G: Graft vessel

### Best Mode for Carrying Out the Invention

Hereinafter, embodiments of the present invention will be described in detail with reference to the drawings. It should be noted that the following descriptions of the preferred embodiments are essentially exemplary and do not intend to limit the present invention or applications and uses thereof.

### <<Embodiment 1>>

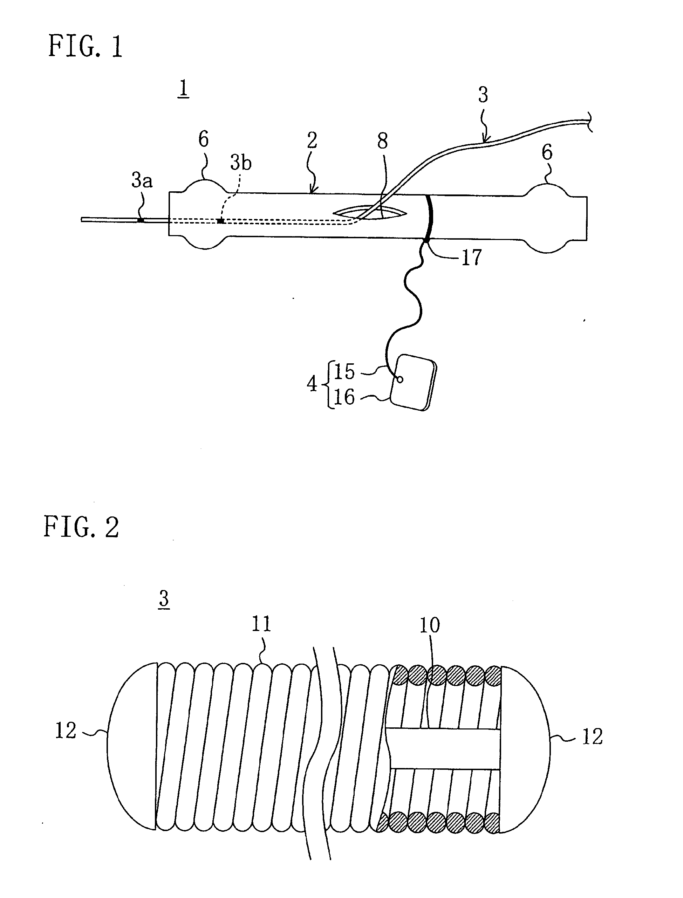

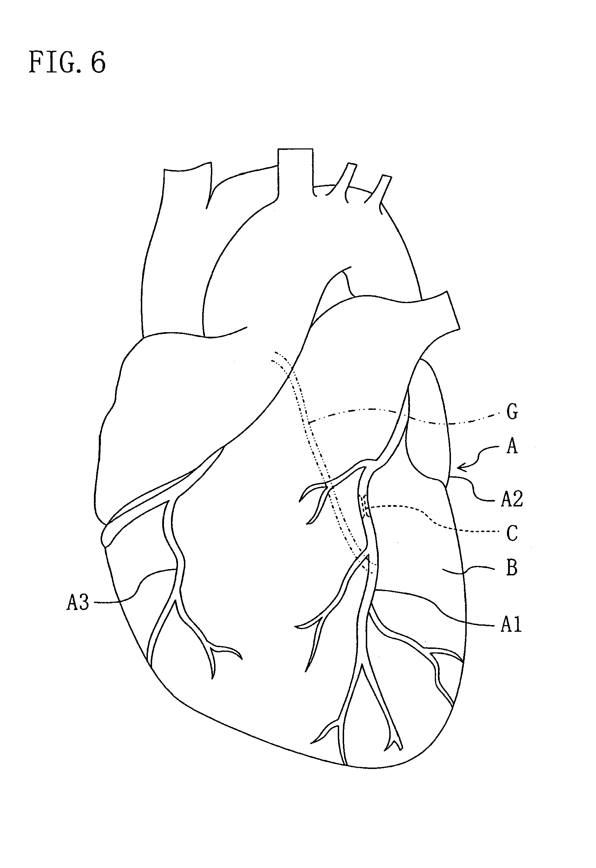

FIG. **1** shows a coronary artery bypass surgery supporting device 1 according to this embodiment of the present invention. This supporting device **1** includes a tubular member **2** which is to be inserted into a coronary artery **A** (shown in FIG.**6**), a guide wire **3** (guide member) for guiding the tubular member **2** into the coronary artery **A**, and a pull member **4** for pulling out the tubular member **2** from the coronary artery **A.**

The tubular member **2** is obtained by shaping a flexible material, such as polyurethane, polyvinyl chloride, or the like, into a straightly extending cylinder. The length of the tubular member **2** is from 13 mm to 20 mm. The outside diameter of the tubular member **2** at a longitudinally intermediate position is from 1.0 mm to 2.6 mm. The length and outside diameter of the tubular member **2** can be set as necessary according to the shape or inside diameter of the coronary artery **A** which is to be subjected to a surgery. The tubular member **2** has tight contact portions **6** at both longitudinal ends. When the tubular member **2** is in the coronary artery A, the tight contact portions **6** are in tight contact with the inner surface of the coronary artery **A.** Each tight contact portion **6** is radially swollen around the whole perimeter of tubular member **2.** Along the tubular member **2** from a longitudinally intermediate position to an end, the outside diameter first increases and then decreases such that the outside diameter at the end is equal to that at the longitudinally intermediate position. Therefore, the outside diameter of the tubular member 2 at the both ends is smaller than that at central part of the tight contact portions **6.**

The longitudinally central part of the tubular member **2** has a slit **8** extending in the longitudinal direction in its peripheral wall. The guide wire **3** is to be inserted through the slit **8.** That is, the slit **8** constitutes an insertion section of the present invention.

The guide wire **3** is formed by an elongated flexible shaft **10** and a **coil 11** wound around the shaft **10** as shown in FIG.**2.** The shaft **10** is formed of, for example, stainless steel, or the like, and has a length of 100 mm to 200 mm. The length of the shaft **10** is equal to the length of the guide wire **3.** The diameter of the shaft **10** is from 0.1 mm to 0.3 mm. The shaft **10** has plugs **12** at the both ends for preventing separation of the coil **11** from the shaft **10.** An end surface of the plug **12** opposite to the coil **11** is curved, such that the inner surface of the coronary artery **A** is not damaged when the guide wire **3** is inserted into the coronary artery **A.** It should be noted that the length of the plug **12** may be arbitrary, for example, about 10 mm.

The coil **11** is formed by single-line winding of a non-splice wire with no gaps. The diameter of the coil **11** is equal to that of the guide wire **3.** The single-line winding of a wire allows the coil **11** to have such a spring property that the coil **11** restores the straight shape. The wire of the coil **11** is formed of, for example, stainless steel, or the like, and has a diameter of 0.01 mm to 0.30 mm. The diameter of the shaft **10,** the diameter of the wire of the coil **11,** and the diameter of the coil **11** are set such that the diameter of the guide wire **3** is from 0.2 mm to 1.0 mm.

The guide wire **3** has such a structure that the elongated shaft **10** is covered with the coil **11** having the spring property, so that the guide wire **3** bent by force restores its original straight shape after the bending force is eliminated.

The shaft **10** is thinner at a first longitudinal end than the other (second) end so that the guide wire **3** is softer at the first end than the second end, and therefore, the first end of the guide wire **3** is readily bendable. This readily bendable portion of the guide wire **3** is from 10% to 20% of the overall length of the guide wire **3,** in this embodiment, from 10 mm to 20 mm. With such a structure that one longitudinal end (first end) of the guide wire **3** is more readily bendable than the other end, the inner surface of the coronary artery **A** is not damaged by the guide wire **3** when the guide wire **3** is inserted into coronary artery **A** with the first end first and the first end comes in contact with the inner surface of the coronary artery **A.** On the other hand, the other longitudinal end (second end) of the guide wire **3** is relatively hard to bend. Therefore, the insertion direction of the first end of the guide wire **3** can readily be controlled by manipulating the guide wire **3** with the other end (second end) in the hand. Such a structure that the first end of the guide wire **3** is readily bendable may be achieved by a design where the first end of the coil **11** has a smaller diameter than the other end, by a design where the wire of the coil **11** has a smaller diameter at the first end than at the other end, or by an arbitrary combination of these designs.

The guide wire **3** is provided with a first marker **3a** around the perimeter in the vicinity of the first end. The guide wire **3** is also provided with a second marker **3b** at a position closer to the other end than the first marker **3a.** The first marker **3a** and the second marker **3b** are to indicate the length of part of the guide wire **3** inserted in the coronary artery **A**. Namely, where the guide wire 3 is inserted into the coronary artery **A** deeper beyond the portion of the second marker **3b,** if the second marker **3b** or the first marker **3a** is observed outside the incision of the coronary artery **A** during a surgery, the operator appreciates that the guide wire **3** is coming out of the coronary artery **A.** In this embodiment, the guide wire **3** is inserted into the coronary artery **A** by about 30 mm. The first marker **3a** is 10 mm away from the first end of the guide wire **3** toward the second end, and the second marker **3b** is 10 mm away from the first marker **3a** toward the second end of the guide wire **3.** The positions of the first marker **3a** and second marker **3b** are arbitrarily determined depending on the overall length and insertion length of the guide wire **3,** the shape of the coronary artery A, etc. The first marker **3a** and second marker **3b** may have different colors. For example, the first marker **3a** is white or blue, while the second marker **3b** is yellow. The first marker **3a** and second marker **3b** may be formed by, for example, electrically or chemically causing corrosion on the surface of the guide wire **3.**

The pull member **4** is formed by a resin thread **15** and a tag **16.** The thread **15** is tied around the outer surface of the tubular member **2.** A knot **17** formed when tied is located at a portion substantially opposite to the slit **8.** Therefore, the thread **15** extends from the portion of the peripheral wall of the tubular member **2** substantially opposite to the slit **8** away from the tubular member **2.** The tag **16** is tied to an end of the thread **15** opposite to the knot **17.**

Next, a procedure of using the coronary artery bypass surgery supporting device **1** having the above-described structure is described. As shown in FIG.**6,** the coronary artery **A** consists mainly of the left anterior descending branch A1 which extends downward across the left anterior area of the cardiac muscle **B,** the left circumflex artery **A2** which extends round to the left posterior area, and the right coronary artery **A3** which extends round to the right posterior area. These vessels have an inside diameter of about 1.0 mm to 2.5 mm. In an example described in this embodiment, the left anterior descending branch **A1** has a stricture **C** formed due to arteriosclerosis, or the like. The tubular member **2** used in this surgery is such that the outside diameter of the longitudinally intermediate part of the tubular member **2** is smaller than the inside diameter of the left anterior descending branch **A1.**

First, an incision **E** for anastomosis of a graft vessel **G** is formed in the distal side of the left anterior descending branch **A1** beyond the stricture **C.** It should be noted that in this embodiment the graft vessel **G** is a so-called free graft made from the large saphenous vein, or the like.

As shown in FIG. **1,** the first end of the guide wire **3** is inserted into the tubular member **2** through the slit **8** of the tubular member **2** so as to protrude out of the opening at the first end of the tubular member **2.** Since the slit **8** of the tubular member **2** is formed by making an incision in its wall, the guide wire **3** is sandwiched by the perimeter of the slit **8** to be held therein, so that the guide wire **3** is prevented from slipping out of the tubular member **2.**

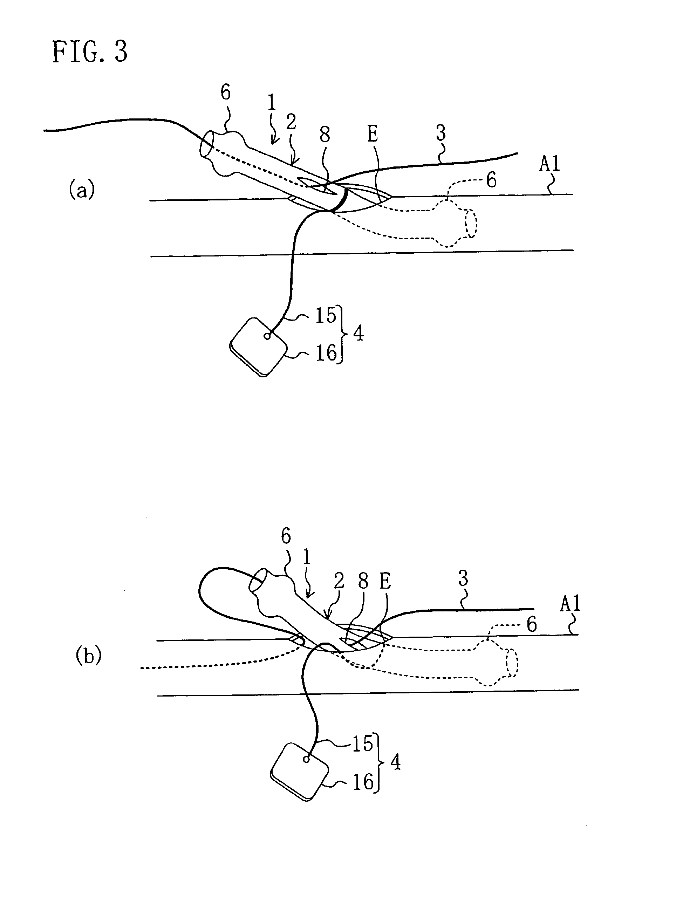

Thereafter, as shown in FIG.**3(a),** the second end of the tubular member **2** is inserted into the left anterior descending branch **A1** through the incision **E** toward the distal side. In this process, the tubular member **2** can readily be inserted because the ends of the tubular member **2** have a smaller diameter than the outside diameter of the tight contact portions **6.**

Then, as shown in FIG.**3(b),** the part of the guide wire **3** protruding out of an opening of the tubular member **2** at the first end is inserted into the proximal side of the left anterior descending branch **A1** through the incision **E.** In this process, the inner surface of the left anterior descending branch **A1** is not damaged by the end of the guide wire **3** because the guide wire **3** is readily bendable as described above. The thread **15** and tag **16** are kept out of the left anterior descending branch **A1** through the incision **E.**

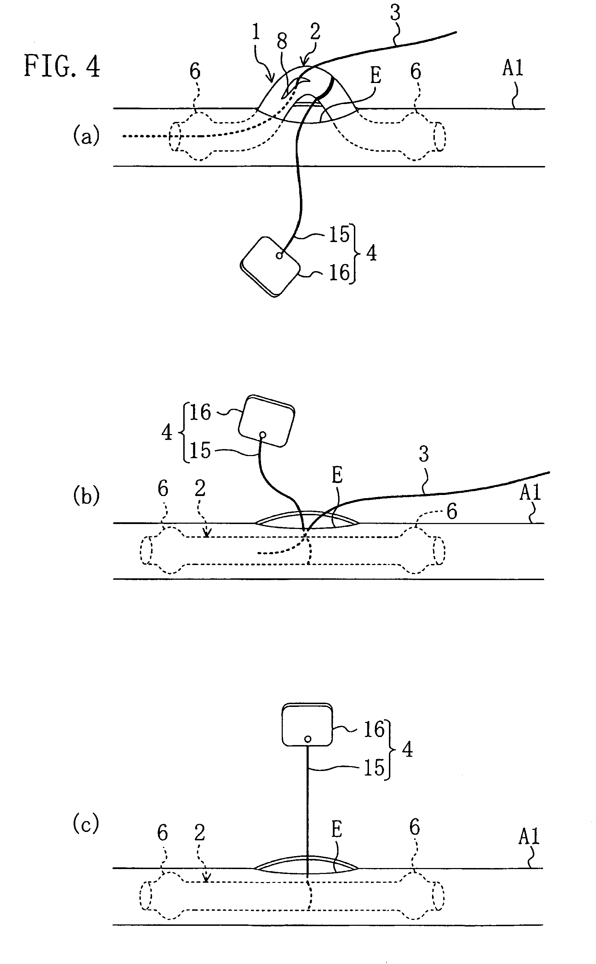

After the guide wire **3** has been inserted into the left anterior descending branch **A1,** the first end of the tubular member **2** is inserted along the guide wire **3** into the proximal side of the left anterior descending branch **A1** as shown in FIG.**4(a).** In this process, the tubular member **2** can readily be inserted because the first end of the tubular member **2** also has a smaller diameter, as can be the second end inserted into the left anterior descending branch **A1.** Then, as shown in FIG.**4(b),** when the tubular member **2** is entirely inserted into the left anterior descending branch **A1,** the outer surfaces of the tight contact portions **6** at the both ends are in tight contact with the inner surface of the left anterior descending branch **A1.** With this structure, blood flowing from the proximal side of the left anterior descending branch **A1** is prevented from leaking through a gap between the inner surface of the left anterior descending branch **A1** and the outer surface of the tubular member **2,** so that almost the total amount of the blood can flow through the tubular member **2.** The blood entering the tubular member 2 flows through the tubular member **2** to the distal side of the left anterior descending branch **A1** so that supply of blood to the cardiac muscle **B** is secured.

Since the tubular member **2** is inserted from the incision **E** side by the guide wire **3,** the guide wire 3 extends from the incision **E** to the outside of the left anterior descending branch **A1** at the time when the insertion is completed. Namely, the slit **8** of the tubular member **2** through which the guide wire **3** is inserted faces the incision **E** side and is in communication with the outside of the left anterior descending branch **A1** through the incision **E.** On the other hand, the knot **17** of the thread **15** faces the cardiac muscle **B** side generally opposite to the incision **E.**

Then, as shown in FIG.**4(c),** the tag **16** is pinched and lightly pulled in such a direction that the tubular member **2** is to be pulled out through the incision **E.** In this process, the force to pull the tag **16** is such that the tubular member **2** does not emerge out of the incision **E.** As a result, since the knot **17** of the thread **15** exists at a portion of tubular member **2** generally opposite to the incision **E,** and the thread **15** extends from the site where the knot **17** is formed, this portion is pulled by the thread **15.** When the portion of the tubular member **2** generally opposite to the slit **8** is pulled, the tubular member **2** axially rotates such that the portion where the knot **17** is formed comes to the incision **E** side, while the slit **8** moves to the cardiac muscle **B** side to be covered with the inner surface of the left anterior descending branch **A1.** With this structure, the blood flowing through the tubular member **2** is unlikely to leak through the slit **8** and, even if some blood leaks, the leaking blood leaks within the left anterior descending branch **A1.** Therefore, the amount of blood leaking outside through the incision **E** is extremely small, so that the observability of the surgery subject field is secured.

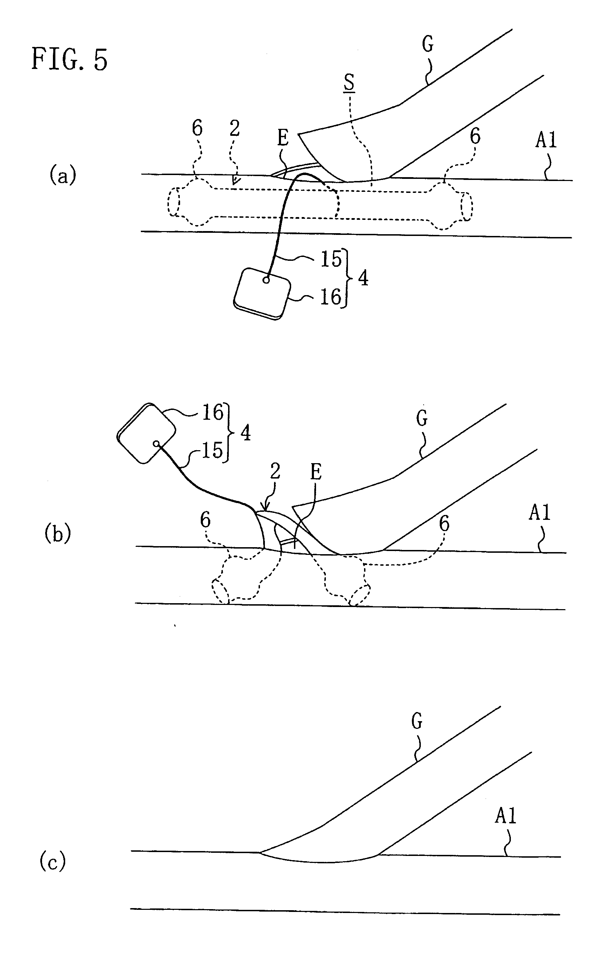

Thereafter, as shown in FIG.**5(a)**, an end of the graft vessel **G** is anastomosed to the periphery of the incision **E** using a sewing needle for anastomosis (not shown). In this process, since the longitudinally intermediate part of the tubular member **2** has a smaller diameter than the inside diameter of the left anterior descending branch **A1,** a gap S occurs between the outer surface of the tubular member 2 and the inner surface of the left anterior descending branch **A1,** in which the sewing needle is inserted and moved. After more than a half of the end of the graft vessel **G** is anastomosed to the left anterior descending branch **A1,** the tag **16** is strongly pulled. By pulling the tag 16, the tubular member **2** is bent at a portion around which the thread **15** is wound and flattened as shown in **FIG.5(b),** and pulled out of the left anterior descending branch **A1** through the incision **E.** In this process, the tubular member **2** can be pulled out of the left anterior descending branch **A1** with smaller force because the tubular member **2** is made of a flexible material, so that invasion to the other tissues is small.

Then, as shown in FIG. **5(c),** the remainder of the graft vessel **G** is anastomosed to the periphery of the incision **E.** In this remaining part of the anastomosis process, blood from the proximal side of the left anterior descending branch **A1** leaks out through the incision **E** because the tubular member **2** has been removed. However, the blood leakage does not substantially deteriorate the observability of the surgery subject field because more than a half of the end of the graft vessel **G** has been anastomosed and hence the amount of leakage is very small.

Therefore, according to the coronary artery bypass surgery supporting device **1** of this embodiment, the tubular member **2** has the slit **8** for insertion of the guide wire **3,** and the tubular member **2** is inserted through the incision **E** of the left anterior descending branch **A1** using the guide wire **3.** Therefore, the tubular member **2** can surely and readily be inserted to a predetermined position. With this, a blood flow to the cardiac muscle **B** is secured during a bypass surgery so that occurrence of a lesion in the cardiac muscle **B** can be avoided.

Further, the tubular member **2** is provided with the pull member **4** extending from a portion of the peripheral wall of the tubular member **2** generally opposite to the slit **8.** Therefore, by pulling, after the tubular member **2** has been inserted into the left anterior descending branch **A1,** the pull member **4** lightly such that the tubular member **2** rotates, the slit **8** is covered with the inner surface of the left anterior descending branch **A1.** As a result, the blood flowing through the tubular member **2** is prevented from leaking from the incision **E** through the slit **8,** so that the observability of the surgery subject field is improved.

The outside diameter of the longitudinally intermediate part of the tubular member **2** is smaller than the inside diameter of the left anterior descending branch **A1,** such that a gap **S** in which a sewing needle is to be moved can be formed between the outer surface of the tubular member **2** and the inner surface of the left anterior descending branch **A1.** Therefore, an anastomosis operation of the graft vessel **G** and the left anterior descending branch **A1** is easy and, hence, the operation time is short.

The tight contact portions **6** are provided at the both ends of the tubular member **2.** After the tubular member **2** is inserted into the left anterior descending branch **A1,** the tight contact portions **6** come into tight contact with the inner surface of the left anterior descending branch **A1.** Therefore, a sufficient amount of blood can be introduced into the tubular member **2** and released to the cardiac muscle **B.** Further, one of the tight contact portions **6** provided at the distal side of the left anterior descending branch **A1** comes into tight contact with the inner surface of the left anterior descending branch **A1,** so that no blood passes through the outside of the tubular member **2** from the distal side toward the longitudinally intermediate part of the tubular member **2.** With this structure, leakage of blood from the incision **E** can be further suppressed, and the observability of the surgery subject field is further improved.

Further, the ends of the tubular member **2** have a smaller diameter than the outside diameter of the tight contact portions **6.** Therefore, the tubular member **2** can readily be inserted into the left anterior descending branch A1.

### <<Embodiment 2>>

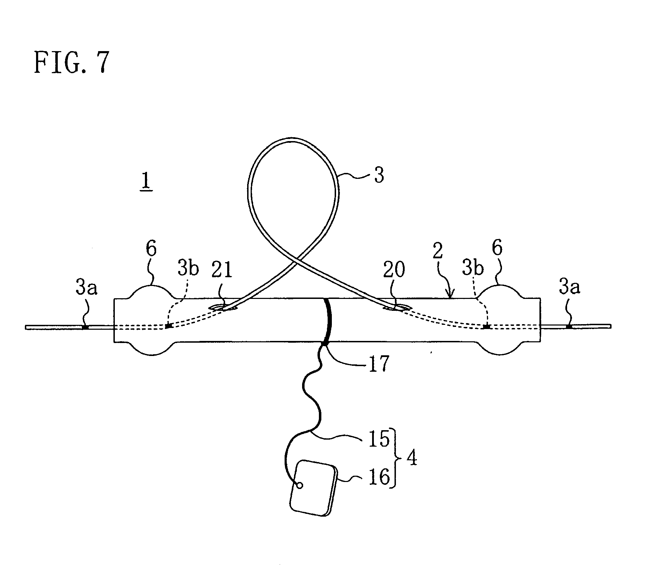

FIG.7 shows a coronary artery bypass surgery supporting device **1** according to embodiment 2 of the present invention. The supporting device of embodiment 2 is substantially the same as that of embodiment 1 except for some aspects in the structure of the tubular member **2.** Thus, hereinafter, elements equivalent to those of embodiment 1 are denoted by the same reference numerals, and the descriptions thereof are omitted.

A tubular member **2** of embodiment 2 has a first slit **20** and a second slit 21 aligned in the longitudinal direction of the tubular member **2** with an interval therebetween. The slits **20** and **21** constitute an insertion section of the present invention as does the slit **8** of embodiment 1. The guide wire **3** is looped between the first slit **20** and the second slit **21.** Both ends of the guide wire **3** are inserted through the first slit **20** and the second slit **21,** so that the guide wire **3** is sandwiched by the perimeters of the slits **20** and **21** to be held therein. Thus, in this embodiment, it is possible to introduce both ends of the tubular member **2** into the left anterior descending branch **A1** using a single guide wire 3.

In embodiment 2, both longitudinal ends of the shaft **10** of the guide wire **3** are thinner than the central part, so that the both longitudinal ends of the guide wire **3** are softer and more readily bendable than the central part. With this structure, when the both ends of the guide wire **3** inserted in the coronary artery **A** come into contact with the inner surface of the coronary artery **A,** damage to the inner surface is prevented. Further, the longitudinally central part of the guide wire **3** exists outside the tubular member **2** and is relatively hard to bend. Therefore, the insertion directions of the both ends of the guide wire **3** can readily be controlled by manipulating the guide wire **3** with the central part in the hand. Such a structure that the both longitudinal ends of the guide wire **3** are more readily bendable than the central part may be achieved by a design where the shape of the coil 11, or the like, is varied in terms of the longitudinal direction as in embodiment **1.**

Next, a procedure of using the coronary artery bypass surgery supporting device **1** having the above-described structure is described. Also in an example described in embodiment **2,** the left anterior descending branch **A1** has a stricture **C** as in embodiment 1.

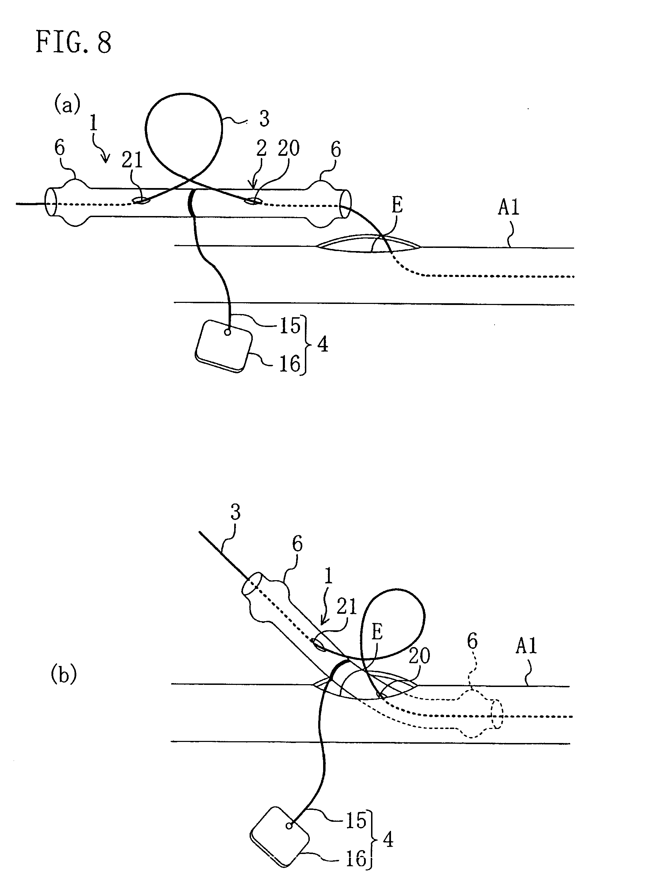

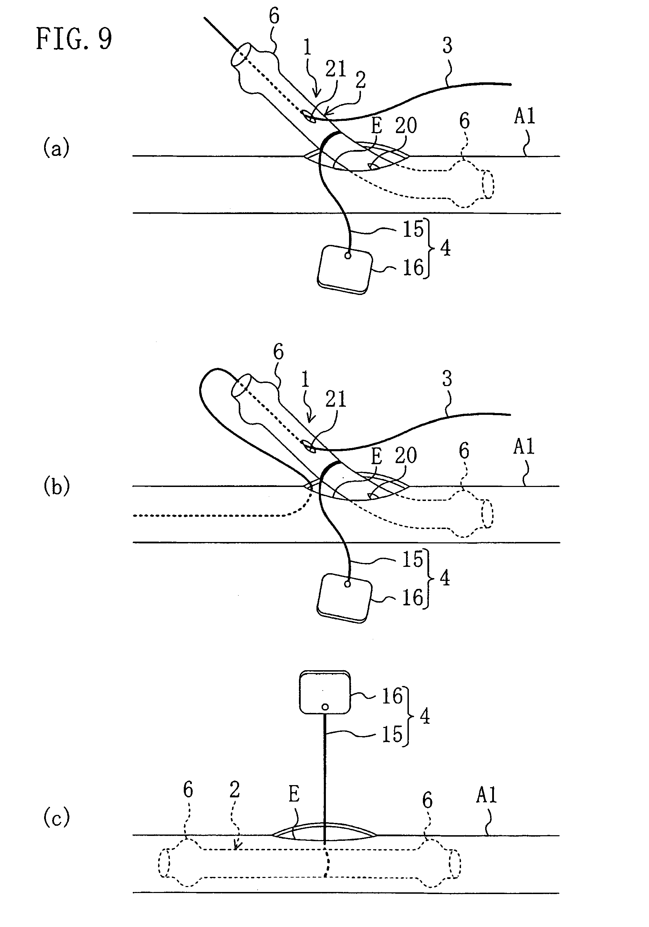

First, as shown in FIG.**8(a),** the end of the guide wire **3** inserted into the first slit **20** is inserted into the distal side of the left anterior descending branch **A1** through the incision **E** of the left anterior descending branch **A1.** Then, as shown in FIG. **8(b),** the end of the tubular member **2** on the first slit **20** side is inserted along the guide wire 3 into the left anterior descending branch **A1.** Thereafter, as shown in FIG.**9(a),** the guide wire 3 is pulled out of the tubular member **2** and the left anterior descending branch **A1.**

Thereafter, as shown in FIG.**9(b),** the other end of the guide wire **3** inserted into the second slit **21** is inserted into the proximal side of the left anterior descending branch **A1** through the incision **E** of the left anterior descending branch **A1.** Then, the end of the tubular member **2** on the second slit **21** side is inserted along the guide wire **3** into the left anterior descending branch **A1.** Thereafter, the guide wire **3** is pulled out of the tubular member **2** and the left anterior descending branch **A1.**

Thereafter, as shown in FIG.**9(c),** the pull member **4** is pulled such that the tubular member **2** rotates as in embodiment **1,** whereby the first slit **20** and the second slit **21** are covered with the inner surface of the left anterior descending branch **A1.**

Thereafter, more than a half of the end of the graft vessel **G** is anastomosed to the left anterior descending branch **A1,** and the tag 16 is strongly pulled to pull the tubular member **2** out of the left anterior descending branch **A1.** Then, the remaining part of the graft vessel **G** is anastomosed to the periphery of the incision **E.**

Thus, according to the coronary artery bypass surgery supporting device **1** of embodiment 2, a blood flow to the cardiac muscle **B** can be secured during a bypass surgery, occurrence of a lesion in the cardiac muscle **B** can be avoided, and the observability of the surgery subject field can be improved as in embodiment 1.

Further, the both ends of tubular member **2** are inserted into the left anterior descending branch **A1** along the guide wire **3.** Therefore, the tubular member **2** can more surely and readily be inserted to a predetermined position in the left anterior descending branch **A1.**

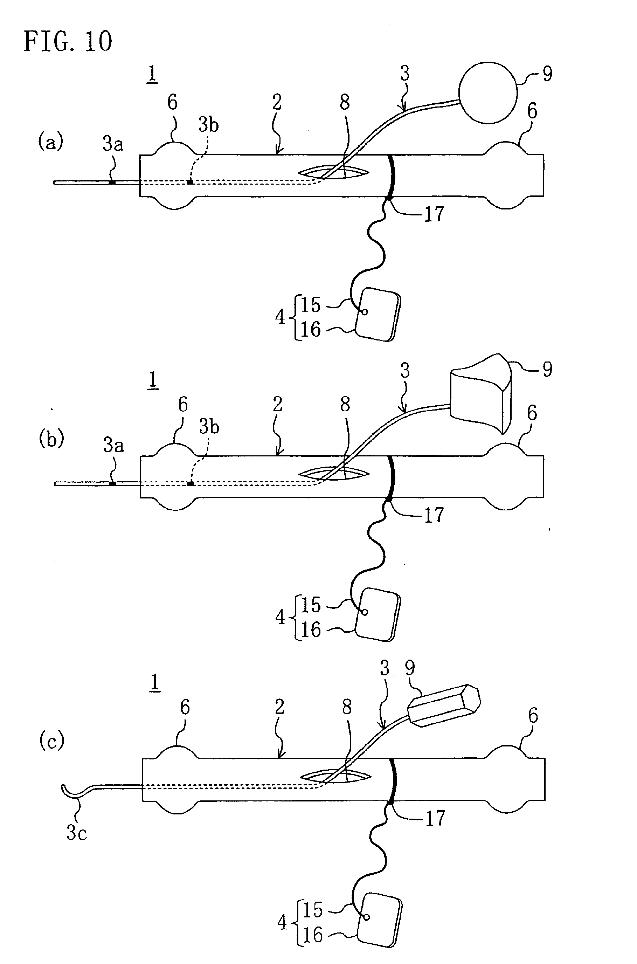

It should be noted that the present invention is not limited to embodiments 1 and 2. For example, as in variation 1 shown in FIG. **10,** a tab **9** made of, for example, a resin material may be provided at the second end of the guide wire **3.** The tab **9** may be formed by molding with the second end of the guide wire **3** inserted therein. Alternatively, the guide wire **3** may be stuck into the tab **9** after the tab **9** is formed. With the tab **9,** the guide wire **3** can readily be manipulated when inserting the guide wire **3** into the coronary artery A. The tab **9** may be a sphere having a diameter of about 10 mm as shown in FIG. **10(a)** or may have such a shape that the width decreases toward the guide wire **3** as shown in FIG. **10(b).** With such a shape that the width of the tab **9** decreases toward the guide wire **3,** the tab **9** is unlikely to slip out of the fingers when the tab **9** is sandwiched on both sides. It should be noted that the shape of the tab **9** is not limited to the above-described examples.

Alternatively, as shown in FIG. **10(c),** the guide wire **3** may have a hook-like curved part **3c** at the first end and a hexagonal tab **9** at the other end. In this case, by axially rotating the tab **9,** the curved part **3c** of the guide wire **3** accordingly rotates, so that the guide wire **3** can readily be inserted into the coronary artery **A.** Since the tab **9** has a hexagonal cross section, the rotation angle of the curved part **3c** can be seen without pulling the guide wire **3** out of the coronary artery **A.** Still alternatively, the tab **9** may have the shape of a quadrangular or triangular prism.

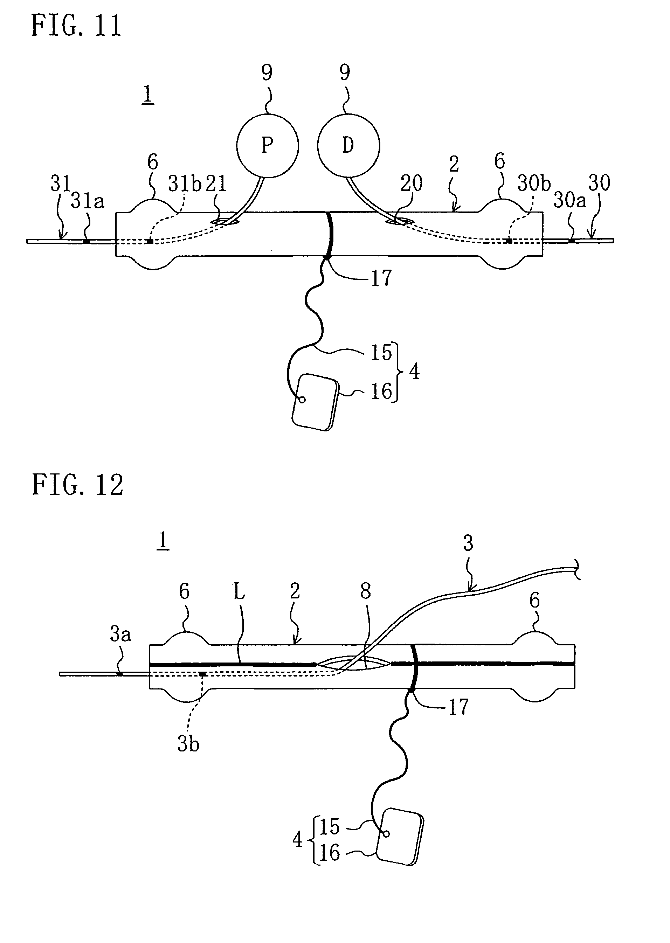

Alternatively, as in variation 2 shown in FIG. **11,** the guide member of the present invention may include a first guide wire **30** for guiding the first end of the tubular member **2** into the distal side of the left anterior descending branch **A1** and a second guide wire **31** for guiding the second end of the tubular member **2** into the proximal side of the left anterior descending branch **A1.** The guide wires **30** and **31** are each provided with a tab **9.** The first guide wire **30** and the second guide wire **31** each have an overall length of 100 mm to 150 mm. The tab **9** of the first guide wire **30** has the initial letter D of "distal", while the tab **9** of the second guide wire **31** has the initial letter P of "proximal". With such two distinguishable guide wires **30** and **31,** even if the first ends of the guide wires **30** and **31** have different shapes, the both ends of tubular member **2** can surely be inserted into the left anterior descending branch **A1** with the help of the corresponding guide wires **30** and **31.** Alternatively, the tabs **9** of the first guide wire **30** and the second guide wire **31** may have different colors or different shapes for distinguishability of the guide wires **30** and **31.**

The guide wire **3** is provided with a first marker **3a** around the perimeter in the vicinity of the first end. The guide wire **3** is also provided with a second marker **3b** at a position closer to the other end than the first marker **3a.** The first marker **3a** and the second marker **3b** are to indicate the length of part of the guide wire **3** inserted in the coronary artery **A.** The first guide wire **30** is provided with a first marker **30a** and a second marker **30b** and the second guide wire **31** is provided with a first marker **31a** and a second marker **31b** as in embodiment 1.

A tubular member **2** of variation 2 has a first slit **20** at the first end side and a second slit **21** at the second end side as in embodiment 2. The first guide wire **30** is inserted through and held by the first slit **20,** and the second guide wire 31 is inserted through and held by the second slit **21.**

In the case of using a coronary artery bypass surgery supporting device **1** according to variation 2, part of the first guide wire **30** protruding out of an opening of the tubular member **2** at the first end is inserted into the distal side of the left anterior descending branch **A1,** and then, the first end of the tubular member **2** is inserted into the left anterior descending branch **A1** along the guide wire **30.** Thereafter, part of the second guide wire **31** protruding out of an opening of the tubular member **2** at the second end is inserted into the proximal side of the left anterior descending branch **A1,** and then, the second end of the tubular member **2** is inserted into the left anterior descending branch **A1** along the guide wire **31.** The first guide wire **30** and the second guide wire **31** may be pulled out through the first slit **20** and the second slit **21,** respectively, at any timing after the first and second ends of the tubular member **2** have been inserted into the left anterior descending branch **A1.**

After the tubular member **2** is inserted into the left anterior descending branch **A1** in this way, the tag **16** is pulled as described above such that the tubular member **2** rotates, and then, anastomosis of the graft vessel **G** is carried out.

As in variation shown in **FIG. 12,** the outer surface of the tubular member **2** may be provided with a line **L** extending in an axial direction. With the line **L** on the tubular member **2,** the slits **8, 20** and **21** can be formed by using a cutter along the line **L.** Thus, formation of the slits **8, 20** and **21** in the tubular member **2** can readily be carried out, and accordingly, the man-hours for producing the tubular member **2** can be reduced. The line **L** may be printed on the tubular member **2** or may be provided by making a groove or ridge in the peripheral wall of the tubular member **2.**

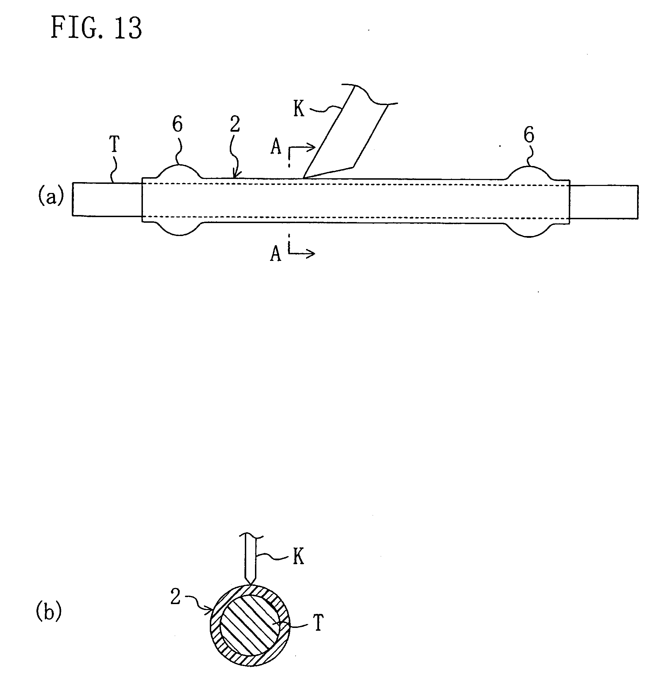

When forming the slits **8, 20** and **21** in the tubular member **2,** a bar **T** made of a resin more rigid than the tubular member **2** may be inserted into the tubular member **2** before the slits are formed in the outer surface of the tubular member **2** by a cutter **K** as shown in FIG. **13.** The outside diameter of the bar **T** is slightly smaller than the inside diameter of the tubular member **2** such that substantially no gap is formed between the bar **T** and the tubular member **2.** With such an arrangement, the tubular member **2** is not flattened when the cutter **K** is applied to the tubular member **2** for forming the slits **8, 20** and **21.** Thus, the cutter **K** can be strongly applied to the tubular member **2,** so that the slits **8, 20** and **21** can readily be formed. Alternatively, the tubular member **2** in which the slits **8, 20** and **21** are not formed may be held together with the bar **T** inserted therein for the purpose of forming the slits **8, 20** and **21** in the tubular member **2** before or after the start of a surgery. Alternatively, the bar **T** may be of a metal material.

Although in embodiments I and 2 the tubular member **2** is first inserted into the distal side of the left anterior descending branch **A1** and then into the proximal side, the tubular member **2** may be first inserted into the proximal side and then into the distal side.

The guide wires **3, 30** and **31** may be, for example, a twine of wires or may be made of a non-metal material, such as resins. Each of the guide wires **3, 30** and **31** may have generally uniform softness across the wire. The guide wire **3** may have three or more markers or may have only one marker. Alternatively, the guide wire **3** may have no marker.

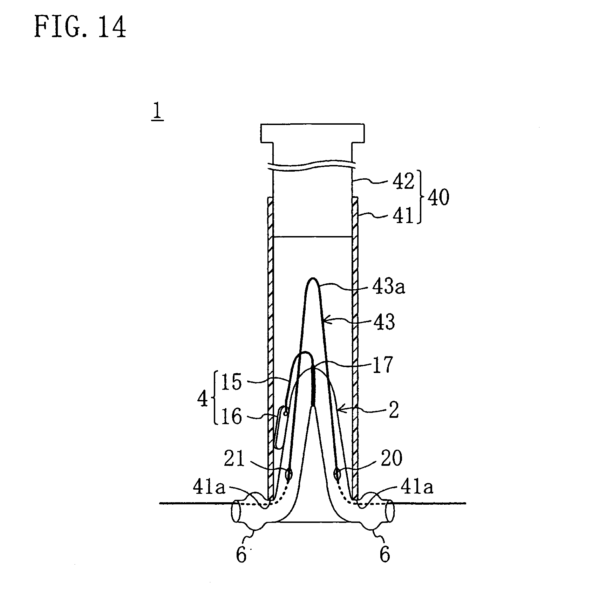

**<<**Embodiment 3>> FIG. **14** through FIG. **19** show a coronary artery bypass surgery supporting device **1** according to embodiment 3 of the present invention. The coronary artery bypass surgery supporting device **1** of embodiment 3 is substantially the same as the supporting devices of embodiments 1 and 2 except for an insertion member **40** which enables easy insertion of the tubular member **2** into the coronary artery. Therefore, in embodiment 3, elements equivalent to those of embodiments 1 and 2 are denoted by the same reference numerals, and the descriptions thereof are omitted.

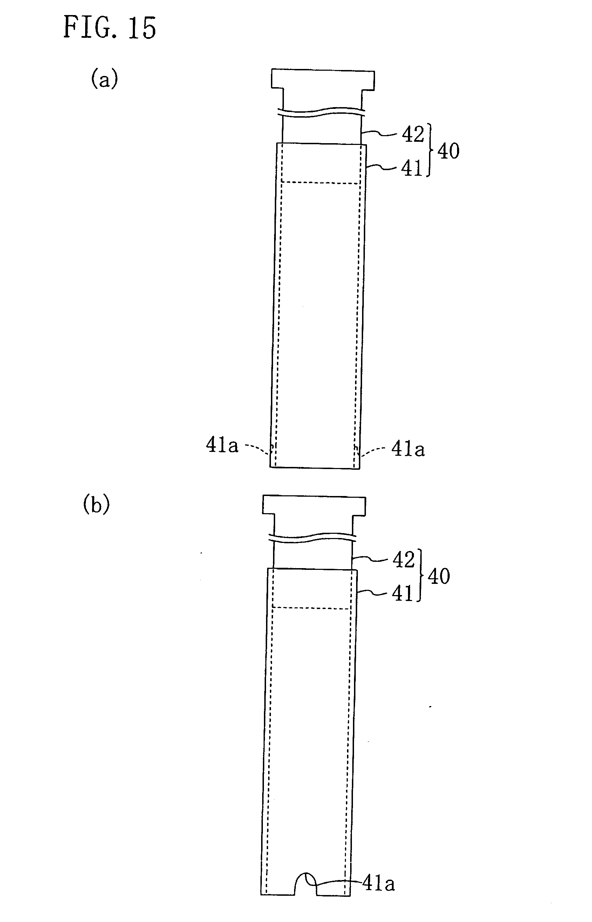

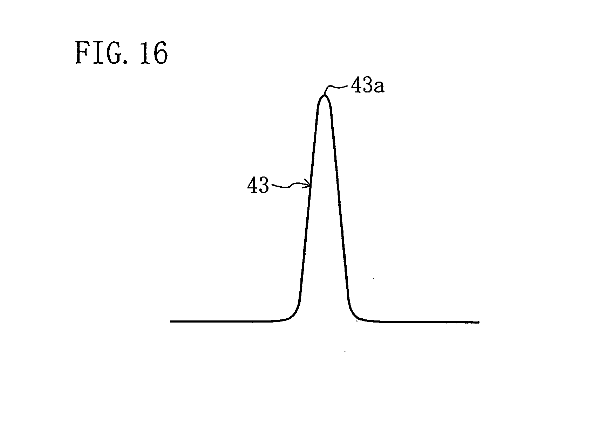

Referring to FIG. **15,** the insertion member **40** includes a generally-circular cylinder **41** and a generally-circular poll **42** which is to be inserted into the cylinder **41.** The first axial end of the cylinder **41** has two notches **41a** each having a generally-U shape, which are separated from each other by about 180° in a perimeter direction. The width of the notch **41a** is substantially equal to the outside diameter of the tubular member **2.** The axial length of the poll **42** is greater than the axial length of the cylinder **41.** Referring to FIG. **16,** the longitudinally central part of a guide wire **43** has a V-shape section **43a** which is bent into a generally-V shape. Parts of the guide wire **43** at both sides of the V-shape section **43a** are bent to extend in opposite directions. A tubular member **2** of embodiment 3 has the same structure as that of the tubular member **2** of embodiment 2.

Next, a procedure of using the coronary artery bypass surgery supporting device **1** of embodiment 3 is described. The ends of the guide wire **43** are inserted into the tubular member **2** through the first slit **20** and the second slit **21** to protrude out of the openings of the tubular member **2** at the first and second ends. At this point in time, the V-shape section **43a** of the guide wire **43** is extending from the first slit **20** and the second slit 21. With the both end parts of the guide wire **43** inserted into the tubular member **2** in this way, the longitudinally central part of the tubular member **2** is bent into a V-form, while the both end parts of the tubular member **2** extend in opposite directions. At this point in time, the separation between the both end parts of the tubular member **2** is greater than the inside diameter of the cylinder **41.**

The tubular member **2** with the guide wire **43** inserted therethrough is inserted into the cylinder **41** from the notch **41a** side. In this process, the both end parts of the tubular member **2** are pinched to be made closer to each other such that the separation between the end parts is smaller than the inside diameter of the cylinder **41.** With such a form, the tubular member **2** is inserted with the V-shape section **43a** first. When entirely inserted, the tight contact portions **6** are outside the notches **41a,** and the both end parts of the tubular member **2** fit in the notches **41a** as shown in FIG. **14.**

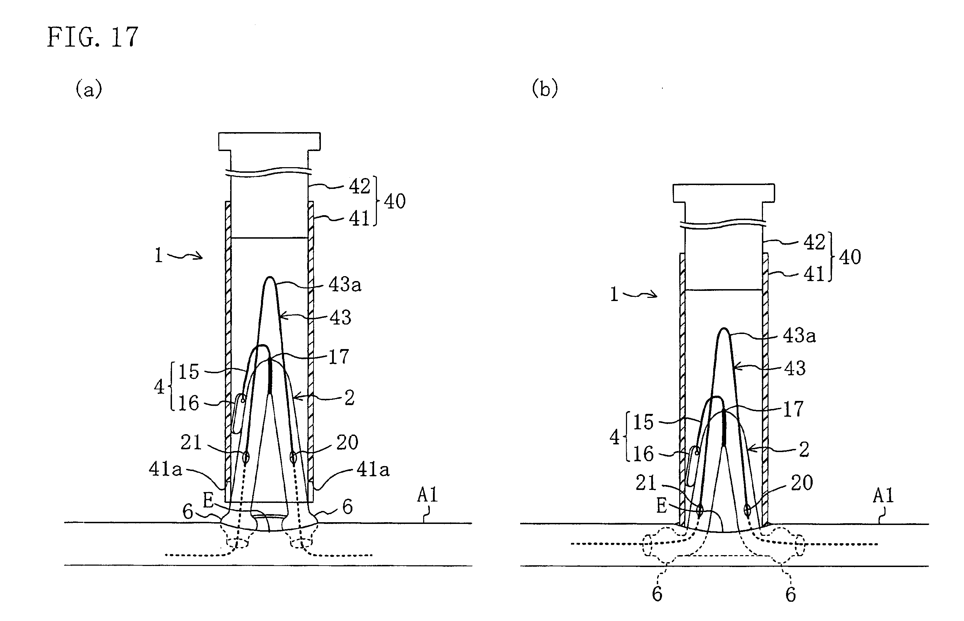

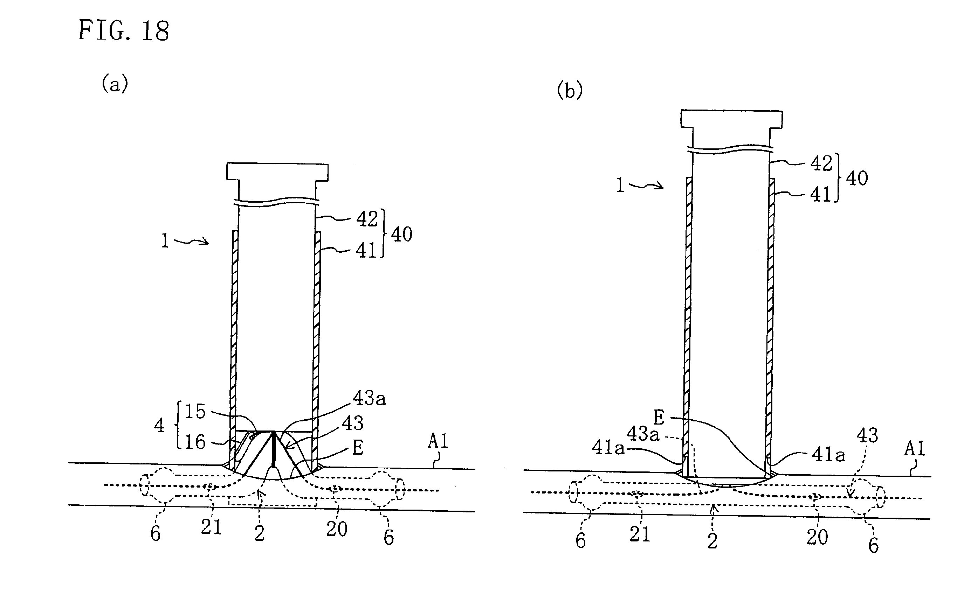

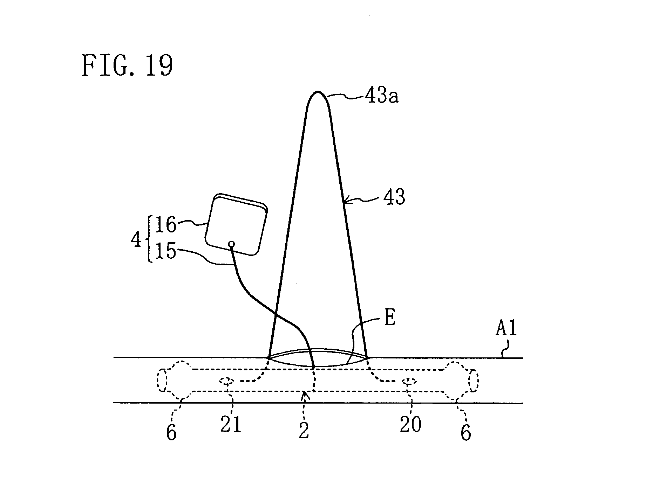

Then, as shown in **FIG.17(a),** the parts of the guide wire **43** protruding out of the tubular member **2** are inserted into the left anterior descending branch **A1** through the incision **E,** and the tight contact portions 6 of the tubular member **2** are inserted into the left anterior descending branch A1 in the same way. Thereafter, as shown in FIG. **17(b),** the notch **41a** side of the cylinder **41** is inserted in the incision **E.** Then, as shown in FIG. **18(a),** the poll **42** is depressed into the cylinder **41,** so that the tubular member **2** and the guide wire **43** are pushed by the end face of the poll **42** into the left anterior descending branch **A1.** At this point in time, the both ends of the tubular member **2** are guided by the guide wire **43** to the distal and proximal sides of the left anterior descending branch **A1.** After the tubular member **2** is entirely inserted into the left anterior descending branch **A1** as shown in FIG. **18(b),** the cylinder **41** is pulled out of the incision **E** as shown in FIG. **19,** and then, the guide wire **43** is pulled out of the tubular member **2** and the left anterior descending branch **A1.** After the guide wire **43** has been pulled out, the tag **16** is pulled such that the tubular member **2** rotates as in embodiment 1, and then, an anastomosis operation of the graft vessel **G** is carried out.

In the above-described embodiments, the coronary artery bypass surgery carried out for the case where the left anterior descending branch **A1** has the stricture **C** has been described. However, the coronary artery bypass surgery supporting device **1** of the present invention is applicable to a coronary artery bypass surgery carried out on the left circumflex artery **A2** or right coronary artery **A3**.

In the above-described embodiments, the coronary artery bypass surgery carried out using the free graft vessel **G** has been described. However, the coronary artery bypass surgery supporting device **1** of the present invention is applicable to a coronary artery bypass surgery carried out using, for example, an internal thoracic artery as the graft vessel.

The pull member **4** may be adhesively fixed to or integrally formed with the peripheral wall of the tubular member **2.** The pull member 4 may be formed only by a flexible member without the accompaniment of the tag **16.**

The insertion section provided in the peripheral wall of the tubular member **2** is not limited to the slits **8, 20** and **21** but may be formed by a hole.

### Industrial Applicability

As described above, a coronary artery bypass surgery supporting device according to the present invention is applicable to, for example, a coronary artery bypass surgery carried out for a case where the left anterior descending branch has a stricture.

## Claims

1. A coronary artery bypass surgery supporting device, comprising:
a flexible tubular member which is to be inserted into a coronary artery of a heart through an incision formed at a portion of the coronary artery for anastomosis of a graft vessel for allowing a blood flow from a proximal side to a distal side of the coronary artery beyond the incision, the tubular member having an insertion section in its peripheral wall;
an elongated guide member which is inserted into the tubular member through the insertion section for guiding an end of the tubular member into the coronary artery for insertion of the tubular member into the coronary artery; and
a pull member for pulling out the tubular member from the coronary artery through the incision, the pull member extending outside the tubular member from a portion of the tubular member generally opposite to the insertion section in a perimeter direction.

2. The coronary artery bypass surgery supporting device of claim 1, wherein a longitudinally intermediate part of the tubular member has an outside diameter smaller than an inside diameter of the coronary artery.

3. The coronary artery bypass surgery supporting device of claim 1 or 2, wherein the tubular member has tight contact portions at both longitudinal ends, the tight contact portions having a diameter greater than an intermediate part of the tubular member such that an outer surface of the tight contact portions comes into tight contact with an inner surface of the coronary artery.

4. The coronary artery bypass surgery supporting device of claim 3, wherein the both longitudinal ends of the tubular member have a diameter smaller than the outside diameter of the tight contact portions.
